# EUROPÄISCHE PATENTSCHRIFT

(11) **EP 0 909 156 B1**
(45) Veröffentlichungstag und Bekanntmachung des Hinweises auf die Patenterteilung: **03.09.2003**
(21) Anmeldenummer: 97929307.3
(22) Anmeldetag: 30.06.1997
(51) Int. Cl.: A61K 7/00, A61K 7/06

(54) **VERWENDUNG VON HYDROGELBILDNERN UND MITTEL ZUR BEHANDLUNG KERATINISCHER FASERN**
USE OF HYDROGEL FORMERS AND AGENTS FOR THE TREATMENT OF KERATINOUS FIBRES
UTILISATION DE GENERATEURS D'HYDROGEL ET AGENTS POUR LE TRAITEMENT DE FIBRES DE KERATINE

(30) Priorität: 01.07.1996 DE 19626141
(43) Veröffentlichungstag der Anmeldung: 21.04.1999
(73) Patentinhaber: Hans Schwarzkopf & Henkel GmbH & Co. KG, 22763 Hamburg (DE)
(72) Erfinder: MÜLLER, Rainer, H., D-12161 Berlin (DE); HERBORT, Jens, D-10825 Berlin (DE); HÖFFGEN, Elisabeth, D-22299 Hamburg (DE); KRÜGER, Marcus, D-22763 Hamburg (DE)
(74) Vertreter: Foitzik, Joachim Kurt
(86) Internationale Anmeldenummer: EP9703394
(87) Internationale Veröffentlichungsnummer: WO98000092

(56) Entgegenhaltungen:
- WO-A-95/04774
- FR-A- 2 670 673

## Beschreibung

Die Erfindung betrifft die Verwendung von Hydrogelbildnern in speziellen Zubereitungen zur Behandlung von keratinischen Fasern sowie solche Zubereitungen.

Die Reinigung und Pflege der Haare sowie die dekorative Gestaltung der Frisur sind wichtige Bestandteile der menschlichen Körperpflege. Entsprechend groß sind die Bemühungen, sowohl dem Friseur als auch dem Endverbraucher in jeder Hinsicht optimierte Produkte zur Verfügung zu stellen. Ein Schwerpunkt der Entwicklungsarbeit liegt dabei auf dem Bemühen, negative Einflüsse der einzelnen Haarbehandlungsverfahren wie Reinigung. Färbung. Blondieren oder Dauerwellen zu minimieren. geschädigtes oder angegriffenes Haar wieder zu reparieren oder möglichen Schädigungen vorzubeugen. Dabei werden sowohl neue Wirkstoffe gesucht als auch verbesserte Konfektionierungsverfahren, die den Effekt des Wirkstoffes am Haar verbessern, die Applikation erleichtern und/oder die Lagerstabilität der entsprechenden Produkte verbessern.

Als eine Möglichkeit zur Bereitstellung verbesserter Produkte wurden in jüngerer Zeit Mittel mit einem Gehalt von Liposomen intensiv untersucht. Liposomen lassen sich als geschlossene, mikroskopische Strukturen charakterisieren, die aus konzentrisch geordneten Amphiphildoppelschichten bestehen, die ihrerseits wäßrige Kompartimente voneinander abtrennen. Die Herstellung von Liposomen ist dem Fachmann bekannt und in einer großen Anzahl von Übersichtsartikeln und Monographien beschrieben. Übliche Methoden sind z.B. die Hochdruckhomogenisation, die Filmmethode, die Ultraschallmethode, die Reverse-Phase-Methode sowie speziell die Infusions-, Injektions-, Lösungsmittel-, Dialyse-, Extrusions- und Sprühtrocknungsmethode.

Liposomen werden insbesondere wegen ihrer Ähnlichkeit zu Zellmembranen seit Jahren als multifunktionelles Träger- und Transportsystem für verschiedenste Substanzen und Wirkstoffe kosmetisch, dermatologisch und pharmazeutisch eingesetzt.

Dabei liegt das Hauptaugenmerk jedoch auf der Wirkung beladener Liposomen an der Haut; zudem müssen diese Liposomen in der Regel zusätzlich z. B. durch Cholesterin, Kohlenhydrate oder Vitamine stabilisiert werden.

Andererseits sind die konditionierende Eigenschaften von bestimmten Liposomenbildnem, insbesondere von Phospholipiden (Lecithinen), am Haar bekannt. Ein häufiges Argument gegen die Verwendung von natürlichen Phospholipiden in der Kosmetik ist deren Empfindlichkeit gegenüber oxidierenden Einflüssen, was insbesondere zu einer ungenügenden Langzeitstabilität führt. Zur Erhöhung der Langzeitstabilität von Phospholipid-Formulierungen wurde daher bereits die Verwendung spezieller Zusätze, z.B. Vitamine, Cholesterin, negativ geladene Phospholipide, hydrogenierte Phospholipide mit gesättigten Acylketten oder Zucker, vorgeschlagen.

In der PCT-Patentanmeldung WO 93/06813 wurde schließlich eine Methode zur konditionierenden Haarbehandlung durch Einsatz und Anwendung von wäßrigen Zubereitungen mit 1 Gew.% Lipidvesikeln zur Reduzierung von Haarspliß beschrieben, wobei insbesondere ein wäßriges Haarkonditionierungsmittel mit einem Gehalt an 0,8 Gew.-% Liposomen auf der Grundlage Leinsamenöl und Wasser unter Verwendung vom Carbomer® 981 (Polyacrylsäure) offenbart wird. Ein Nachteil ist, daß gemäß der Lehre dieser Anmeldung keine Haarbehandlungsmittel mit einem Gehalt von über 1 Gew.-% an Liposomen hergestellt werden können.

Aufgabe der vorliegenden Erfindung ist es daher, ein Haarbehandlungsmittel zur Verfügung zu stellen, welches die Haare langanhaltend gepflegt erscheinen läßt, die Trocken- und Naßkämmbarkeit sowie den Griff des nassen und trockenen Haares deutlich verbessert und welches lagerstabil ist. Weiterhin soll sich das Haarbehandlungsmittel nicht negativ, wenn möglich sogar positiv, auf die Kopfhaut auswirken..

Es wurde nunmehr überraschenderweise gefunden, daß die Aufgabe durch wäßrige Mittel mit liposomalen Strukturen, die Hydrogelbildner enthalten, in hervorragender Weise gelöst werden kann.

Ein erster Gegenstand der Erfindung ist daher die Verwendung von Hydrogelbildnern in wäßrigen Mitteln mit liposomalen Strukturen zur Behandlung keratinischer Fasern.

Unter keratinischen Fasern oder Keratinfasem werden erfindungsgemäß Pelze, Wolle, Federn und insbesondere menschliche Haare verstanden.

Gemäß einer ersten Ausführungsform werden erfindungsgemäß anionische Hydrogelbildner eingesetzt.

Unter den anionischen Hydrogelbildnern sind Polymere mit Carboxylat- oder Sulfonatgruppen bevorzugt. Dabei kann es sich sowohl um Homo- als auch um Copolymere handeln. Entsprechende Verbindungsklassen sind beispielsweise unvernetzte, teilvernetzte und vernetzte Polyacrylsäuren sowie Acrylsäure-Methacrylsäure-Copolymere.

Innerhalb der Gruppe der anionischen Polymeren haben sich die Copolymeren, die mindestens ein nichtionogenes Monomer enthalten, als erfindungsgemäß besonders geeignet erwiesen. Bevorzugte nichtionogene Comonomere sind Acrylamid, Methacrylamid, Acrylsäureester, Methacrylsäureester, Vinylpyrrolidon und Vinylester. Bevorzugte anionische Copolymere sind Acrylsäure-Acrylamid-Copolymere, sowie Polyacrylamidcopolymere mit Sulfonsäuregruppen-haltigen Monomeren. Ein besonders bevorzugtes anionisches Copolymer besteht aus 70 bis 55 Mol% Acrylamid und 30 bis 45 Mol% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt.

Gemäß einer zweiten Ausführungsform werden als Hydrogelbildner nichtionogene Polymere eingesetzt. Erfindungsgemäß bevorzugte nichtionogene Polymere sind Polyacrylamid, Polymethacrylamid, Polyvinylacetat, Polyvinylalkohol sowie Copolymere aus den genannten Monomeren. Besonders bevorzugte nichtionogene Polymere sind Polyacrylamid, Polymethacrylamid sowie Copolymere aus diesen beiden Monomeren. Ganz besonders bevorzugt ist Polyacrylamid.

Die genannten anionischen und nichtionischen Polymeren sind unter einer Vielzahl von Bezeichnungen als Handelsprodukte erhältlich, z.B. Carbopol®, Latekoll®, Pemulen®, Acrysol® und Sepigel®. Erfindungsgemäß als Hydrogelbildner einsetzbar sind aber auch Naturprodukte und deren Derivate wie Agar-Agar, Guar-Gum, Alginate, Xanthan-Gum und Celluloseether wie Hydroxyalkylcellulosen.

Der Hydrogelbildner kann auch in Form eines, bevorzugt wäßrigen, Compounds vorliegen, das noch weitere Komponenten enthalten kann.

Ein erfindungsgemäß ganz besonders bevorzugter Hydrogelbildner ist das Handelsprodukt Sepigel®305 der Firma SEPPIC. Dieses Compound enthält neben der Polymerkomponente gemäß Deklaration C₁₃-C₁₄-Isoparaffin und Laureth-7.

Hydrogelbildner auf Basis von Polyacrylamiden, seien sie nun anionisch oder nichtionogen, haben sich als besonders geeignet für Mittel mit einem hohen Elektrolytgehalt erwiesen; bei Mitteln mit hohem Elektrolytgehalt kann bei Verwendung anderer Hydrogelbildner die Viskositätsstabilität in einigen Fällen nicht voll befriedigen.

Die erfindungsgemäße Lehre umfaßt auch die gleichzeitige Verwendung von zwei oder mehr der oben genannten Hydrogelbildner.

Die erfindungsgemäßen Zusammensetzungen enthalten zwingend liposomale Strukturen. Die Liposomen sind bevorzugt auf Basis von Phospholipiden, Sphingolipiden und/oder Cerobrosiden aufgebaut.

Gemäß einer bevorzugten Ausführungsform enthalten die Liposomen als wesentlichen oder ausschließlichen Baustoff Phospholipide. Phospholipide sind entweder Ester des Glycerins oder des Sphingosin mit Fettsäuren (insbesondere gesättigten und/oder ungesättigten Fettsäuren mit 12 bis 24 Kohlenstoffatomen, wie Palmitinsäure, Stearinsäure, Ölsäure, Linolsäure und Linolensäure) und einem Phosphatidrest. Erfindungsgemäß bevorzugt sind die Phospholipide auf Glycerin-Basis wie Lecithine (Phosphatidyl-Cholin), Kephaline (Phosphatidyl-Ethanolamine), Phosphatidyl-Serine und Phosphatidyl-Inositol. Ebenfalls erfindungsgemäß verwendbar sind die Sphingolipide wie Sphingomyeline und Cerobroside. Ceramide sind ebenfalls als Basis für die liposomalen Strukturen in den erfindungsgemäßen Mitteln verwendbar.

Wenngleich erfindungsgemäß sowohl tierische (z.B. Eilecithin) als auch pflanzliche Phospholipide verwendet werden können, so sind doch die pflanzlichen Phospholipide bevorzugt. Besonders geeignete pflanzliche Phospholipide sind Sojalecithin, Rapslecithin, Erdnußlecithin, Sonnenblumenlecithin und Baumwollsaatöllecithin. Innerhalb dieser Gruppe ist das Sojalecithin bevorzugt.

Erfindungsgemäß bevorzugte Sojalecithine enthalten als Fettsäurereste, bezogen auf das Sojalecithin, 5 bis 50 Gew.-%, vorzugsweise 10 bis 25 Gew.-%, besonders bevorzugt 15 bis 23 Gew.-%, Palmitinsäurereste, 0-15 Gew.-%, vorzugsweise 2 bis 6 Gew.-%, Stearinsäurereste, 0 bis 20 Gew.-%, vorzugsweise 5 bis 15 Gew.-%, besonders bevorzugt 7 bis 13 Gew.-%, Ölsäurereste, 20 bis 100 Gew.-%, vorzugsweise 40 bis 70 Gew.-%, besonders bevorzugt 50 bis 65 Gew.-%, Linolsäurereste und 1 bis 20 Gew.-%, vorzugsweise 2 bis 15 Gew.-%, besonders bevorzugt 3 bis 10 Gew.-%, Linolensäurereste, wobei die Summe dieser Mengenangaben jeweils 100 % nicht überschreitet.

Ebenfalls bevorzugt sind Sojalecithine, die, jeweils bezogen auf das Sojalecithin, 5 bis 40 Gew.-%, vorzugsweise von 18 bis 26 Gew.-%, Phosphatidylcholin, 2 bis 30 Gew.-%, vorzugsweise von 10 bis 18 Gew.-%, Phosphatidylethanolamin und 0 bis 25 Gew.-%, vorzugsweise von 8 bis 14 Gew.-%, Phosphatidylinosit enthalten.

Erfindungsgemäß besonders geeignete Phospholipide sind auch der Publication No. 14 der Firma Lucas Meyer, Liposomes - The Pro-Liposome Approach, Jean-Pierre-Arnaud, September 1993, zu entnehmen, auf die ausdrücklich Bezug genommen wird.

Ein für die erfindungsgemäße Verwendung ganz besonders geeignetes Sojalecithin ist das Handelsprodukt Emulmetik® 300 der Fima Lucas Meyer (s. Produktdatenblatt Nr. 030694/300).

Die Liposomen können auch aus mehreren der vorgenannten Phospholipide aufgebaut sein.

Die Herstellung der Liposomen erfolgt bevorzugt mittels Hochdruckhomogenisation bei einem Druck von 500 bis 1500 bar und einem Durchlauf von 1 bis 10 Zyklen, vorzugsweise 3 bis 5 Zyklen. Es sind jedoch prinzipiell auch alle anderen, dem Fachmann bekannten Methoden einsetzbar.

Ebenfalls Gegenstand der Erfindung sind wäßrige Mittel mit liposomalen Strukturen zur Behandlung von keratinischen Fasern, die einen Hydrogelbildner nach einem der Ansprüche 2 bis 8 enthalten und bei denen die Liposomen aus Phospholipiden, Spbingolipiden und/oder Ceramiden aufgebaut sind. Dabei sind Mittel bevorzugt, die pflanzliche Phospholipide als Liposomenbildner enthalten.

Die Hydrogelbildner sind in den erfindungsgemäßen Mitteln bevorzugt in Mengen von 0,01 bis 10 Gew.-%, insbesondere von 0,1 bis 10 Gew.-% und ganz besonders bevorzugt von 0,4 bis 7 Gew.-%, bezogen auf das gesamte Mittel, enthalten. Insbesondere bei Hydrogelbildnern auf Basis von Poylacrylsäuren mit einem Molekulargewicht von 350000 bis 5000000 g/mol können Mengen zwischen 1 und 10 Gew.-%, bezogen auf das gesamte Mittel, bevorzugt sein.

Bezogen auf das gesamte Mittel sind die Liposomen bevorzugt in Mengen von 1 bis 20 Gew.-% enthalten. Mengen von 1 bis 10 Gew.-%, insbesondere von 2 bis 4 Gew.-%, sind besonders bevorzugt. Entsprechend der Art des Haarbehandlungsmittels können auch Liposomenmengen von 5 bis 15 Gew.-%, bezogen auf das Mittel, enthalten sein.

Die Liposomen haben in den erfindungsgemäßen Mitteln bevorzugt eine mittlere Größe im Bereich von 50 bis 1000 nm, insbesondere im Bereich von 70 bis 300 nm. Sie sind in dem gebrauchsfertigen Mittel unter üblichen Lagerbedingungen über einen Zeitraum von 3 Jahren als stabil zu bezeichnen, d.h. die maximale Größe der Liposomen nach Abschluß der Lagerzeit überschreitet nicht den Wert von 500 nm, und insbesondere von 300 nm.

Die erfindungsgemäßen Mittel weisen bevorzugt einen pH-Wert zwischen 2 und 8 auf. Je nach Art des Mittels können pH-Wert-Bereiche zwischen 4 und 7 bzw. zwischen 5,5 und 6,9 bevorzugt sein.

Die erfindungsgemäßen Mittel enthalten neben den zwingenden Komponenten Wasser, Hydrogelbildner und Liposomenbildner weiterhin bevorzugt mindestens eine Ölkomponente. Bei dieser Ölkomponente handelt es sich bevorzugt um einen Kohlenwasserstoff, ein synthetisches Silikon oder ein natürliches pflanzliches oder tierisches Öl.

Geeignete Kohlenwasserstoffe sind Paraffinöle, insbesondere Mischungen aus gesättigten, linearen und/oder verzweigten Kohlenwasserstoffen sowie Squalan. Innerhalb der Kohlenwasserstoffe sind lineare und/oder gesättigte Kohlenwasserstoffe mit 10-22 Kohlenstoffatomen bevorzugt.

Geeignete Silikonöle sind Dimethylpolysiloxane, Methylphenylpolysiloxane, cyclische Dimethylsiloxane, Siloxane mit quartären Ammoniumgruppen sowie die unter den Bezeichnungen Amodimethicone, Trimethylsilylamodimethicone und Dimethicone Copolyl bekannten Verbindungen. Lineare und cyclische Dimethylpolysiloxane sowie Dimethicon Copolyole sind bevorzugte Öle. Entsprechende Handelsprodukte sind beispielsweise Dow Corning® 344 Fluid, Dow Corning® 345 Fluid sowie Dow Corning® 190, 193 und 939. Der Anteil der Silikone beträgt bevorzugt 0,1 bis 3 Gew.-%, insbesondere 0,5 bis 1,5 Gew.-%, bezogen auf das gesamte Mittel.

Erfindungsgemäß geeignete tierische Öle sind Nerzöl, Purcellinöl, Emuöl, Walrat und Spermöl. Purcellinöl kann bevorzugt sein.

Erfindungsgemäß bevorzugte pflanzliche Öle sind Weizenkeimöl, Mandelöl Macadamianußöl, Sonnenblumenöl, Babassuöl, Avocadoöl, Olivenöl, Traubenkernöl und Nachtkerzenöl (candle light oil). Jojobaöl ist innerhalb dieser Gruppe besonders bevorzugt.

Die Ölkomponente kann den erfindungsgemäßen Mitteln separat zugegeben werden. Es ist aber auch möglich, sie in Form eines Compounds zusammen mit dem Hydrogelbildner zuzugeben. Sepigel® 305 enthält neben dem Hydrogelbildner beispielsweise noch ca. 20 Gew.-% C₁₃-C₁₄-Isoparaffinkohlenwasserstoffe.

Die erfindungsgemäßen Mittel enthalten gemäß einer weiteren bevorzugten Ausführungsform als weiteren Bestandteil mindestens einen haaravivierenden und konditionierenden Wirkstoff.

Als avivierende und konditionierende Wirkstoffe kommen bevorzugt kationische Polymere in Betracht. Dies sind in der Regel Polymere, die ein quartäres Stickstoffatom. beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- Polysiloxane mit quatemären Gruppen, wie sie unter den Bezeichnungen Dow Corning® Q2-7224 und 929 sowie Abil®-Quat 3270 und 3272 im Handel erhältlich sind,
- Polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele fiir solche kationischen Polymere.
- Copolymere des Vinylpyrrolidons mit quaternierten Derivaten des Dialkylaminoacrylats- und -methacrylats, wie beispielsweise mit Diethylsulfat quatemierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
- quaternierte Guar-Derivate, wie sie unter den Bezeichnungen Cosmedia Guar® und Jaguar® im Handel erhältlich sind,
- kationisch derivatisierte Proteinhydrolysate, insbesondere kationisch derivatisierte Keratinhydrolysate.
sowie die unter den Bezeichnungen
- Polyquaternium 2,
- Polyquaternium 17,
- Polyquaterniurn 18 und
- Polyquaternium 27
bekannten Polymeren mit quartären Stickstoffatomen in der Polymerhauptkette.

Als avivierende Wirkstoffe geeignete Silikonöle sind insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 193, DC 200, Q2-5220 und DC 1401 vertriebenen Produkte. Geeignete Aminogruppen-haltige bzw. quaternierte Silikone sind beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), Dow Corning®939, SM-2059 (Hersteller: General Electric) und SLM-55067 (Hersteller: Wacker).

Ebenfalls als avivierende Wirkstoffe geeignet sind Proteinhydrolysate, insbesondere Elastin-, Kollagen-, Keratin-, Milcheiweiß-, Sojaprotein-, Mandelprotein-, Seidenprotein-, Kartoffelprotein-, Haferprotein-, Erbsenprotein-, Maisprotein- und Weizenproteinhydrolysate, deren Kondensationsprodukte mit Fettsäuren sowie quaternisierte Proteinhydrolysate.

Gleichsam als avivierende Komponente verwendbar sind quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid sowie Imidazoliniumverbindungen wie die Handelsprodukte Rewoquat® W 75 und W 75PG. Weiterhin können die sehr gut biologisch abbaubaren quatemären Esterverbindungen, wie beispielsweise die unter dem Warenzeichen Stepantex® vertriebenen Dialkylammoniummethosulfate und Methyl-hydroxyalkyl-dialkoyloxyalkyl-ammoniummethosulfate und die entsprechenden Produkte der Dehyquart®-Reihe verwendet werden. Alternativen dazu stellen die Alkylamidoamine, insbesondere Fettsäureamidoamine wie das unter der Bezeichnung Tego Amid®S 18 erhältliche Stearylamido-propyldimethylamin, dar.

Schließlich können als avivierende Wirkstoffe auch Ampho-Polymere verwendet werden. Unter dem Oberbegriff Ampho-Polymere sind amphotere Polymere, d.h. Polymere, die im Molekül sowohl freie Aminogruppen als auch freie -COOH- oder -SO₃H-Gruppen enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymeren zusammengefaßt, die -COOH- oder -SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z.B. Acryl- und Methacryl-säure), kationisch derivatisierten ungesättigten Carbonsäuren (z.B. Acrylamidopropyl-trimethyl-ammoniumchlorid) und gegebenenfalls weiteren ionischen oder nichtionogenen Monomeren zusammen, wie sie beispielsweise aus der deutschen Offenlegungsschrift 39 29 973 und dem dort zitierten Stand der Technik zu entnehmen sind.

Die avivierenden Wirkstoffe sind in den erfindungsgemäßen Mitteln bevorzugt in Mengen von etwa 0,01 bis 2 Gew.-%, bezogen auf das gesamte Mittel, enthalten, im Falle der Silikonöle insbesondere in Mengen von 0,1 bis 2 Gew.-%, im Falle der kationischen Polymeren insbesondere in Mengen von 0,01 bis 1 Gew.-%.

Gemäß einer ersten bevorzugten Ausführungsform sind die Liposomen in den erfindungsgemäßen Mitteln unbeladen.

Gemäß einer zweiten bevorzugten Ausführungsform sind die Liposomen in den erfindungsgemäßen Mitteln mit mindestens einem kosmetischen Wirkstoff beladen.

Ein erster bevorzugter kosmetischer Wirkstoff, mit dem die Liposomen beladen sein können, ist Panthenol oder ein Panthenolderivat wie Panthothensäure oder ein Ester. Panthenol oder seine Derivate sind dann bevorzugt in einer Menge von 0,1 bis etwa 5 Gew.-%, bezogen auf das gesamte Mittel, enthalten. Die Kombination Panthenol(-derivat)/Silikonöl/kationisches Polymer kann in den erfindungsgemäßen Mitteln bevorzugt sein.

Eine zweite Wirkstoffgruppe, mit denen die Liposomen in den erfindungsgemäßen Mitteln beladen sein können, stellen quartäre Ammoniumverbindungen dar. Bezüglich der einzelnen und bevorzugten Vertreter wird auf das oben gesagte verwiesen.

Eine weitere Wirkstoffgruppe stellen die Vitamine dar. Bevorzugte Vertreter sind Vitamin A und dessen Ester wie das Palmitat, Vitamin C, Vitamin E und seine Ester, Vitamine der D und F-Gruppe sowie die Vitamine B₃, B₆ und B₁₂.

Pflanzenextrakte und Allantoin sind ebenfalls Verbindungen, mit denen die Liposomen in den erfindungsgemäßen Mitteln beladen sein können.

Die Art des Haarbehandlungsmittels unterliegt keinen prinzipiellen Beschränkungen. Bevorzugte Mittel sind beispielsweise Haarkuren, Haarspülungen, Haarbalsame, Haarconditioner, Rinses, Haartonics und Haarwässer, Shampoos, Frisiercremes, Haargele und Pomaden. Die Anwendung in Dauerwellmitteln (Wellotionen, Fixiermittel), Haarfärbemitteln, Haartönungsmitteln, Haarfestigern, Haarsprays oder Haarspitzenfluids ist jedoch gleichfalls möglich. Entsprechend dem Verwendungszweck können die Zubereitungen als Lösungen, Emulsionen, Gele, Cremes, Aerosole oder Lotionen formuliert werden.

Abhängig von der Art des Mittels können die erfindungsgemäßen Mittel alle in diesen Mitteln üblichen Bestandteile enthalten. Solche üblichen Bestandteile sind:
- anionische Tenside, wie lineare Fettsäuren (Seifen), Ethercarbonsäuren, Amidethercarboxylate, Acylsarcoside, Acyltauride, Acylisethionate, Sulfobemsteinsäuremono- und dialkylester, lineare Alkansulfonate, lineare Alpha-Olefinsulfonate, Alpha-Sulfofettsäuremethylester sowie insbesondere Alkylsulfate und Alkylpolyglykolethersulfate und Kokosmonoglyceridsulfate, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- Nichtionogene Tenside, wie Anlagerungsprodukte von Ethylenoxid und/oder Propylenoxid an lineare Fettalkohole, an Fettsäuren und an Alkylphenole, Fettsäuremono- und -diester von Anlagerungsprodukten von 1 bis 30 Mol Ethylenoxid an Glycerin sowie insbesondere C₈-C₂₂-Alkylmono- und -oligoglycoside und deren ethoxylierte Analoga
- ampholytische und zwitterionische Tenside, wie die sogenannten Betaine,
- nichtionische Polymere wie beispielsweise Vinylpyrrolidon/Vinylacrylat-Copolymere, Polyvinylpyrrolidon und Vinylpyrrolidon/Vinylacetat-Copolymere,
- anionische Polymere, wie Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden, Polyoxycarbonsäuren, wie Polyketo- und Polyaldehydocarbonsäuren und deren Salze, sowie Polymere und Copolymere der Crotonsäure mit Estern und Amiden der Acryl- und der Methacrylsäure, wie Vinylacetat-Crotonsäure- und Vinylacetat-Vinyl-propionat-Crotonsäure-Copolymere, Vinylacetat/Butylmaleat/Isobornylacrylat-Copolymere und Methylvinylether/Maleinsäureanhydrid und deren Ester,
- Verdickungsmittel wie Gelatine und Pektine,
- Strukturanten wie Glucose und Maleinsäure,
- Parfümöle, Dimethylisosorbid und Cyclodextrine,
- Lösungsvermittler wie Ethanol, Isopropanol, Ethylenglykol, Propylenglykol, Glycerin und Diethylenglykol sowie Anlagerungsprodukte von Ethylenoxid an Rizinusöle / gehärtete Rizinusöle und ethoxylierte Fettalkohole,
- Farbstoffe,
- Antischuppenwirkstoffe wie Piroctone Olamine, Zink Omadine und Climbazol,
- Substanzen zur Einstellung des pH-Wertes,
- Lichtschutzmittel wie (Sulfonsäure-) Derivate von Benzophenon und Ester der Zimtsäure,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole und Diole wie Propandiol,
- Trübungsmittel wie Latex oder Styrol/Acrylamid-Copolymere,
- Wirkstoffe wie Bisabolol,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Komplexbildner wie EDTA, NTA, β-Alanindiessigsäure und Phosphonsäuren,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Perlglanzmittel wie Ethylenglykolmono- und -distearat, PEG-3-distearat oder VP/Styrol-Copolymerisate,
- direktziehende Farbstoffe
- sogenannte Kuppler- und Entwicklerkomponenten als Oxidationsfarbstoffvorprodukte,
- Aminosäuren und deren Derivate wie Serin, Lysin, Cystein und Glycin sowie
- Konservierungsmittel wie p-Hydroxybenzoesäureester, Benzoe- und Sorbinsäure, 1,2-Dibrom-2,4-dicyanobutan und/oder 2-Phenoxyethanol
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Treibmittel wie Propan-Butan-Gemische, N₂O, Dimethylether, CO₂ und Luft sowie
- Antioxidantien.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologen-verteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.
Gemäß einem bevorzugten Verfahren werden die erfindungsgemäßen Haarbehandlungsmittel hergestellt, in dem 1 bis 20 Gewichtsteile Phospholipid in 80 bis 99 Gewichtsteilen Wasser gequollen und gemischt werden. Danach wird diese wäßrige Zubereitung durch Homogenisationsbehandlung unter Hochdruck in Liposomen überführt und das so erhaltene Liposomen-Konzentrat in der gewünschten Menge in das Haarbehandlungsmittel eingearbeitet.

Die Anwendung der erfindungsgemäßen Haarbehandlungsmittel erfolgt in üblicher Weise entsprechend dem Typ des Haarbehandlungsmittel. So werden nach einer ersten Ausführungsform Haarbehandlungsmittel, die Haarkuren oder Haarspülungen darstellen, üblicherweise für eine Einwirkzeit von 1 bis 30 Minuten auf die behandelten Haare aufgebracht und anschließend ausgespült. Dabei wird im Falle der Spülungen die Einwirkzeit eher im kürzeren Bereich, d.h. bei ca. 2 bis 5 Minuten, liegen, während im Falle von Haarkuren eine längere Einwirkzeit, d.h. Zeiten zwischen 10 und 30 Minuten, insbesondere zwischen 20 und 30 Minuten, bevorzugt sind.

Selbstverständlich können die Haarbehandlungsmittel, insbesondere die Haarkuren, auch als sogenannte "leave-on"-Produkte auf dem Haar belassen werden.

Die nachfolgenden Beispiele sollen die erfindungsgemäße Lehre weiter erläutern.

### Beispiele

### 1. Verfahren

### 1.1. Herstellung der Liposomen-Suspension

Die Herstellung der Liposomen erfolgte mittels Hochdruckhomogenisation mit einem Micron LAB 40 high pressure homogenizer (für kleine Mengen) oder mit einem Micron LAB 60 high pressure homogenizer (Mengen von mehreren Litern) der Fa. APV Gaulin, Lübeck, Deutschland. Dazu wurden 10 g Emulmetik® 300 in 90 g Wasser gegeben und fiir 5 Minuten quollen gelassen. Anschließend rührte man das gequollene Material vorsichtig mit einem Glasstab. Danach wurde es mit einem Ultra-Turrax-Gerät bei 13500 U/min vordispergiert. Anschließend wurde diese Vordispersion im Hochdruckhomogenisator bei 1000 bar und mindestens 3 Zyklen zu Liposomen verarbeitet.

### 1.2. Bestimmung der Naßkämmkraft

Die Bestimmung der Naßkämmkraft erfolgte an Haarsträhnen, die eine Länge von ca. 10 cm, eine Breite von 1,5 cm und ein Gewicht zwischen 2 und 3 g hatten. Die Haarsträhnen wurden durch Behandlung mit einem handelsüblichen Blondiermittel (Igora Vario Blond Plus, Schwarzkopf, gemäß Gebrauchsanweisung mit 9 Gew.-%iger Wasserstoffperoxid versetzt) je nach gewünschtem Schädigungsgrad bis zu 40 min lang gezielt vorgeschädigt. Nach dem Ausspülen des Blondiermittels, Shampoonieren des Haares und erneutem Spülen wurde die Naßkämmkraft ein ersten Mal bestimmt (Nullwert; oB = ohne Behandlung).

Danach ließ man 0,5 g der zu untersuchenden Mischung 5 Minuten lang auf die Strähne einwirken. Anschließend wurden die Haarsträhnen mit lauwarmem Wasser gründlich gespült und die Naßkämmkraft erneut bestimmt (mB = mit Behandlung) .

Die Bestimmung der Kämmkräfte, die in einem Bereich von 50 bis 400 mN lagen, erfolgte mit einer Universalprüfmaschine vom Typ UTS 2 der Firma SCHENCK-UTS Prüftechnik Vertriebs GmbH, D-64293 Darmstadt, Deutschland.

Die unter Punkt 3.1. angegebenen Zahlenwerte sind Mittelwerte aus jeweils 60 Messungen. Die Signifikanz der aufgeführten Werte war in allen Fällen mindestens 95 %.
Weiterhin ist eine Differenz von 5 % zwischen den Werten vor und nach der Behandlung als signifikant anzusehen.

### 1.3. Gebrauchsprüfungen

### 1.3.1. Verwendung als Haarkur

Je nach Haarlänge wurden 10 bis 20 g des zu untersuchenden Mittels in das zuvor shampoonierte und handtuchgetrocknete Haar einmassiert und dort für ca. 20 Minuten belassen. Nach dieser Einwirkzeit wurde das erfindungsgemäße Mittel gründlich mit lauwarmem Wasser ausgespült. Anschließend wurde das Haar getrocknet und gewünschtenfalls wie gewohnt weiterfrisiert bzw. -behandelt.

### 1.3.2. Verwendung als Haarspülung

Je nach Haarlänge wurden 10 bis 20 g des zu untersuchenden Mittels 1 in das zuvor shampoonierte, handtuchgetrocknete Haar einmassiert und dort für ca. 3 Minuten belassen. Nach der angegebenen Einwirkzeit wurde das erfindungsgemäße Mittel gründlich mit lauwarmem Wasser ausgespült. Anschließend wurde das Haar getrocknet und gewünschtenfalls wie gewohnt weiterfrisiert bzw. -behandelt.

### 1.3.3. Halbseitentests

Diese Bestimmungen wurden an Testpersonen mit bis zu 20 cm langen, normalen oder leicht porösen Haaren durchgeführt, bei denen die Haare auf der linken und der rechten Kopfseite gleichgut kämmbar waren.
Dazu wurde der Haarschopf der Testperson durch einen Mittelscheitel geteilt. Die zu testenden Produkte wurden auf die eine Hälfte des shampoonierten, handtuchtrockenen Haares aufgetragen, während das Referenzprodukt auf das Haar der anderen Kopfhälfte aufgetragen wurde. Es wurden gleiche Produktmengen und Einwirkzeiten für die beiden Schopfhälften eingehalten. Nach dem Ausspülen der Produkte wurde die überschüssige Feuchtigkeit von den Haaren abgetupft. Die Schopfhälften wurden dann, jede Schopfhälfte einzeln, mit einem speziellen Kamm feucht durchgekämmt. Danach wurden die Strähnen der jeweiligen Halbseiten für die Beurteilung des Griffes zwischen Daumen und Zeigefinger genommen, bewegt und schließlich ließ man sie durch die Finger gleiten. Anschließend wurde das Haar 10 bis 15 Minuten (je nach Haarlänge) mit einer Trockenhaube getrocknet. Die Schopfhälften wurden nach vorangegangener, gleicher Behandlung trocken durchgekämmt. Nach dem Kämmen der Schopfhälften (je 1 Kamm für eine Schopfhälfte) wurden die Strähnen der Halbseiten für die Beurteilung des Griffes zwischen Daumen und Zeigefinger genommen, bewegt und schließlich durch die Finger gleiten gelassen.

### 2. Untersuchte Mischungen

### 2.1. Zusammensetzung

Die Zusammensetzungen der untersuchten Mischungen sind in der folgenden Tabelle aufgerührt [alle Mengenangaben, soweit nicht anders angegeben, sind Gewichtsteile].

| Mischung | B1 | B2 | V1 | V2 | V3 | V4 |
|---|---|---|---|---|---|---|
| Sepigel®305¹ | 3,5 | 3,5 | 3,5 | 3,5 | - | 3,5 |
| Emulmetik®300^{2,}³ | 3,0 | 3,0 | - | - | 3,0 | - |
| Emulmetik®300^{2,}⁴ | - | - | - | 3,0 | - | - |
| Euxyl®K 400⁵ | 0,2 | 0,2 | 0,2 | 0,2 | 0,2 | 0,2 |
| Merquat®550⁶ | - | 0,1 | - | - | - | 0,1 |
| Dow Corning®939⁷ | - | 0,75 | - | - | - | 0,75 |
| Panthenol⁸ | - | 0,65 | - | - | - | 0,65 |
| Wasser (entsalzt) | <--------------------- ad 100 ------------------------> | | | | | |

| | | | | | | |
|---|---|---|---|---|---|---|
| ¹ Compound mit der INCI-Bezeichnung "Polyacrylamide (and) C₁₃-C₁₄ Isoparaffin (and) Laureth-7" (SEPPIC) | | | | | | |
| ² Sojalecithin (INCI-Bezeichnung: Lecithin) (LUCAS MEYER) | | | | | | |
| ³ eingebracht als Liposomen, hergestellt mittels Hochdruckhomogenisation | | | | | | |
| ⁴ direkt zugesetzt | | | | | | |
| ⁵ 1,2-Dibrom-2,4-dicyanobutan/Phenoxyethanol (INCI-Bezeichnung: Methyldibromo glutaronitril (and) Phenoxyethanol) (SCHÜLKE & MAYR) | | | | | | |
| ⁶ Polymerisat aus Dimethyldiallylammoniumchlorid und Acrylamid (8 % Aktivsubstanz in Wasser; INCI-Bezeichnung: Polyquaternium-7) (CHEMVIRON) | | | | | | |
| ⁷ Compound aus einem aminofunktionellen Silikon, Poly(12)ethylen-tridecylalkohol und Hexadecyltrimethylammoniumchlorid (INCI-Bezeichnung: Amodimethicone (and) Trideceth-12 (and) Cetrimonium Chloride) (DOW CORNING) | | | | | | |
| ⁸ 75 Gew.-%ige wäßrige Lösung | | | | | | |

Die Vergleichsbeispiele V1 und V4 enthielten den Hydrogelbildner, nicht aber die Liposomen-bildende Komponente, so daß keine liposomalen Strukturen vorlagen. Vergleichsbeispiel V2 enthielt die Liposomen-bildende Komponente Lecithin in einer Form, die eine Liposomenbildung ausschloß. Dem Vergleichsbeispiel V3 fehlte der Hydrogelbildner.

### 2.2. Herstellung

### 2.2.1. Mischung B1

3,5 Gewichtsteile Sepigel®305 wurden in einem Becherglas vorgelegt und 66,3 Gewichtsteile Wasser, in dem 0,2 Gewichtsteile Euxyl®K 400 gelöst worden waren, langsam unter Rühren mit einem Dissolver-Rührer zugegeben. Die Rührgeschwindigkeit wurde soweit erhöht, daß eine ausreichende Durchmischung gewährleistet war. Dann wurden 30 Gewichtsteile der zuvor hergestellten 10 Gew.-%igen wäßrigen Liposomen-Suspension dem Ansatz langsam zugegeben. Anschließend wurde solange gerührt, bis eine homogene Formulierung vorlag. Die Mischung hatte eine Viskosität von 17500 mPas bei 20°C, gemessen mit einem Haake Rotovisco RV 20 Gerät.

### 2.2.2. Mischung B2

A) 3,5 Gewichtsteile Sepigel®305 wurden in einem Becherglas vorgelegt und 45 Gewichtsteile Wasser, in dem 0,2 Gewichtsteile Euxyl®K 400 gelöst worden waren, langsam unter Rühren mit einem Dissolver-Rührer zugegeben. Die Rührgeschwindigkeit wurde dabei soweit erhöht, daß eine ausreichende Durchmischung gewährleistet war.
B) In 19,8 Gewichtsteilen Wasser wurden 0,1 Gewichtsteile Merquat®550, 0,75 Gewichtsteile Dow Corning®939 und 0,65 Gewichtsteile Panthenol (75 Gew.-%ige wäßrige Lösung) separat in einem Becherglas unter Rühren mit einem Magnetrührer gelöst.
Die Lösung B) wurde langsam unter Rühren zum Ansatz A) gegeben. Dem Gemisch wurden dann 30 Gewichtsteile der zuvor hergestellten 10 Gew.-%igen wäßrigen Liposomen-Suspension langsam zugegeben. Anschließend wurde so lange gerührt, bis eine homogene Formulierung entstand.

### 2.2.3. Mischung V1

3,5 Gewichtsteile Sepigel®305 wurden in einem Becherglas vorgelegt und 96,3 Gewichtsteile Wasser, in dem 0,2 Gewichtsteile Euxyl®K 400 gelöst worden waren, langsam unter Rühren mit einem Dissolver-Rührer zugegeben. Die Rührgeschwindigkeit wurde soweit erhöht, daß eine ausreichende Durchmischung gewährleistet war.

### 2.2.4. Mischung V2

3,5 Gewichtsteile Sepigel®305 wurden in einem Becherglas vorgelegt und 66,3 Gewichtsteile Wasser, in dem 0,2 Gewichtsteile Euxyl®K 400 gelöst worden waren, langsam unter Rühren mit einem Dissolver-Rührer zugegeben. Die Rührgeschwindigkeit wurde soweit erhöht, daß eine ausreichende Durchmischung gewährleistet war.
30 Gewichtsteile einer zuvor hergestellten 10 Gew.-%igen wäßrigen Emulmetik®300-Lösung, der das Emulmetik 300 direkt zugesetzt worden war, wurden dem Ansatz langsam zugegeben. Anschließend wurde so lange gerührt, bis eine homogene Formulierung entstanden war.

### 2.2.5. Mischung V3

69,8 Gewichtsteile Wasser wurden in einem Becherglas vorgelegt. Unter Rühren mit einem Propeller-Rührer wurden Gewichtsteile Euxyl K®400 zugegeben.
30 Gewichtsteile der zuvor hergestellten 10 Gew.-%igen wäßrigen Liposomen-Suspension wurden dem Ansatz langsam zugegeben. Anschließend wurde so lange gerührt, bis eine homogene Formulierung entstanden war.

### 2.2.6. Mischung V4

A) 3,5 Gewichtsteile Sepigel®305 wurden in einem Becherglas vorgelegt und 45 Gewichtsteile Wasser, in dem 0,2 Gewichtsteile Euxyl®K 400 gelöst worden waren, langsam unter Rühren mit einem Dissolver-Rührer zugegeben. Die Rührgeschwindigkeit wurde soweit erhöht, daß eine ausreichende Durchmischung gewährleistet war.
B) In 19,8 Gewichtsteile Wasser wurden 0,1 Gewichtsteile Merquat®550, 0,75 Gewichtsteile Dow Corning®939 und 0,65 Gewichtsteile Panthenol (75 Gew.-%ige wäßrige Lösung) separat in einem Becherglas unter Rühren mit einem Magnetrührer gelöst.
Lösung B) wurde langsam unter Rühren zum Ansatz A) gegeben.
Anschließend wurde so lange gerührt, bis eine homogene Formulierung vorlag..

### 3. Ergebnisse

### 3.1. Naßkämmkraft

Die bei der Bestimmung der Naßkämmkraft mit wenig vorgeschädigten Haaren erhaltenen Ergebnisse sind in der folgenden Tabelle 1 zusammengestellt. Die Werte sind in mN angegeben.

**Tabelle 1**

| **Mischung** | **B1** | **V1** | **V2** |
|---|---|---|---|
| Naßkämmkraft oB | 177,00 | 181,25 | 172,50 |
| Naßkämmkraft mB | 162,50 | 170,50 | 164,25 |
| Naßkämmkrafterniedrigung | 14,50 | 10,75 | 8,25 |

Die Erniedrigung der Naßkämmkraft war somit fiir Haare, die mit der erfindungsgemäßen Mischung B1 behandelt worden waren, deutlich höher als für Haare, die mit einer der Vergleichsmischungen V1 oder V2 behandelt worden waren.

Die bei der Bestimmung der Naßkämmkraft mit stark vorgeschädigten Haaren erhaltenen Ergebnisse sind in der folgenden Tabelle 2 zusammengestellt. Die Werte sind in mN angegeben.

**Tabelle 2**

| **Komponenten** | **B1** | **B2** | **V3** |
|---|---|---|---|
| Naßkämmkraft oB | 369,38 | 370,25 | 357,51 |
| Naßkämmkraft mB | 302,36 | 253,70 | 320,96 |
| Kämmkrafterniedrigung | 67,02 | 116,55 | 36,55 |

Die Erniedrigung der Naßkämmkraft betrug bei Verwendung der erfindungsgemäßen Mischungen 22 % (B1) bzw. 46 % (B2), während sie bei Verwendung der Vergleichsmischung (V3) lediglich 10 % betrug.

### 3.2. Gebrauchsprüfungen

### 3.2.1. Verwendung der Mischungen als Haarkur

Mit der erfindungsgemäßen Mischung B behandeltes normales bis leicht poröses Haar ließ sich im nassen und trockenen Zustand leicht durchkämmen und der Griff des Haares war deutlich angenehmer als vor der Anwendung des erfindungsgemäßen Mittels. Das behandelte Haar sah gesund und gepflegt aus.

Die Verbesserung der Kämmbarkeit von in gleicher Weise mit den Mischungen V2 bzw. V3 behandelten Haaren war sowohl im nassen als auch im trockenen Zustand deutlich geringer; die Haare waren schwerer zu entwirren. Der Griff der Haare, die mit diesen Vergleichsmischungen behandelt wurden, war weniger geschmeidig; die Haare sahen auch weniger gepflegt aus.

Bei Personen mit normalem und leicht porösem Haar wurde festgestellt, daß sich auch das mit einem erfindungsgemäßen Mittel gemäß B2 behandelte Haar sowohl im nassen als auch im trockenen Zustand deutlich besser durchkämmen ließ. Der Griff der so behandelten Haare war angenehm, und die Haare luden sich nicht statisch auf. Bei einer Langzeitanwendung über sechs Wochen (zwei- bis dreimal wöchentlich) war eine Glättung der Haaroberfläche zu beobachten. Das Haar wurde restrukturiert und sah deutlich gesünder und gepflegter aus als vor Beginn der Anwendung des Mittels. Die mit Mitteln gemäß Beispielen V3 und V4 behandelten Haare ließen sich dagegen deutlich schwerer im nassen und trocknen Zustand durchkämmen. Das Haar war schwerer zu entwirren und lud sich statisch auf. Der Griff der mit diesen Mitteln behandelten Haare war deutlich weniger geschmeidig; die Haare sahen weniger gepflegt aus.

### 3.2.2. Halbseitentests

### 3.2.2.1. Mischung B1 / Mischung V2 - poröses Haar

Durch Anwendung der Mischung B 1 wurde die Naß- und Trockenkämmbarkeit des Haares sowie insbesondere der Griff im trockenen Zustand im Vergleich zur Anwendung des Mittels V2 verbessert.

### 3.2.2.2. Mischung B1 / Mischung V2 - normales Haar

Durch Anwendung der Mischung B 1 wurde deutlich die Kämmbarkeit des Haares sowie der Griff des Haares im nassen und trockenen Zustand verbessert. Die trockenen Haare luden sich beim Kämmen oder Bürsten nicht statisch auf. Die mit der Mischung V2 behandelten Haare ließen sich dagegen deutlich schwerer im nassen und trockenen Zustand durchkämmen. Im trockenen Zustand wurde beim Kämmen statische Aufladung mit Sträuben der Haare beobachtet. Außerdem war der Griff der trockenen Haare deutlich weniger geschmeidig.

### 3.2.2.3. Mischung B1 / Mischung V1 - normales Haar

Bei der mit der Mischung gemäß Beispiel B 1 behandelten Haarhälfte wurde eine verbesserte Trockenkämmbarkeit des Haares sowie ein verbesserter Griff im nassen und trockenen Zustand im Vergleich zu der mit der Mischung V1 behandelten Haarhälfte gefunden.

### 3.2.2.4. Mischung B2 / Mischung V4 - normales Haar

Die Anwendung der Mischung B2 verbesserte die Naß- und Trockenkämmbarkeit des Haares und den Griff im nassen und trockenen Zustand erheblich auf der entsprechenden Haarhälfte. Bei der Behandlung der Haare mit der Mischung V4 wurde eine deutlich geringere Verbesserung der überprüften Eigenschaften festgestellt.

### 3.3. Stabilitätstests

Die Stabilität der Liposomen wurde in den erfindungsgemäßen Mischungen B 1 und B2 sowie in Gelen auf der Basis Polyacrylsäure (z.B. Carbopol® 940) überprüft. Die zu überprüfenden Formulierungen wurden unter verschiedenen Bedingungen (-20°C, 4°C, 45°C, Raumtemperatur unter Tageslichtbestrahlung; Raumtemperatur unter Ausschluß des Tageslichts) über einen Zeitraum von 3 Monaten in einem verschlossenen Glas gelagert. Monatlich wurde die Partikelgröße und -verteilung mittels einer photonenkorrelationsspektroskopischen (PCS-)Messung und einer Zeta-Potential-Messung überprüft.

Die Liposomen erwiesen sich in dem Gel auf Basis Polyacrylsäure als äußerst stabil. Sowohl der mittlere Durchmesser als auch die Breite der Verteilung blieben konstant. Lediglich bei der Lagertemperatur von -20°C wurde eine Veränderung der Teilchengröße beobachtet. Dies kann einerseits auf eine Ausdehnung des wasserhaltigen Kerns beim Einfrieren und einer dabei ablaufenden Zerstörung der Phospholipid-Membran und andererseits auf eine Koaleszenz der Liposome, hervorgerufen durch kristallisierendes Wasser in noch nicht erstarrten Bereichen, zurückzuführen sein. Hohe Elektrolytgehalte in der Mischung haben sich als ungünstig für die Stabilität der Mittel auf Basis Polyacrylsäure, insbesondere was die Viskosität betraf, erwiesen.

Die Liposomen in den erfindungsgemäßen Mischungen B 1 und B2 erwiesen sich ebenfalls unter den genannten Bedingung als stabil. Bezüglich Teilchengrößen und -verteilung wurde lediglich ein geringes, für Verkaufsprodukte akzeptierbares Anwachsen der Teilchengröße bei zunehmender Lagerungsdauer und Lagertemperatur beobachtet. Die liposomalen Strukturen wurden dabei mittels rasterelektronenmikroskopischen Aufnahmen nachgewiesen. Ein Vorteil dieser Mischungen auf Basis des Handelsproduktes Sepigel®305 war der äußerst geringe Einfluß auch großer Elektrolytmengen auf die Stabilität und insbesondere die Viskosität der Mischungen.

### 4. Anwendungsrezepturen

Alle Mengenangaben sind Gewichts-%.

### 4.1. Haartonic, auf dem Haar verbleibend

| | |
|---|---|
| Carbopol®940⁹ | 0,25 |
| Jaguar®HP 120¹⁰ | 0,10 |
| Euxyl®K 400 | 0,30 |
| Neutrol®TE¹¹ | 0,42 |
| Lipos®E 300 10%¹² | 3,00 |
| Dow Corning®939 | 0,05 |
| Panthenol⁸ | 0,10 |
| α-Tocopherol¹³ | 0,20 |
| Extrapon®Brennessel Spezial¹⁴ | 1,00 |
| Allantoin¹⁵ | 0,05 |
| Wasser, entsalzt | ad 100 |

| | |
|---|---|
| ⁹ Polyacrylsäure (INCI-Bezeichnung: Carbomer) (GOODRICH) | |
| ¹⁰ 2-Hydroxypropylguar (INCI-Bezeichnung: Hydroxypropyl Guar) (RHONE-POULENC) | |
| ¹¹ N,N,N',N'-Tetrakis-(2-hydroxypropyl)-ethylendiamin (INCI-Bezeichnung: Tetrahydroxypropyl Ethylenediamine) (BASF) | |
| ¹² Liposomenzubereitung auf Basis Sojalecithin (Emulmetik®300) mit 10 Gew.-% Liposomengehalt (LUCAS MEYER) | |
| ¹³ (MERCK) | |
| ¹⁴ aufbereiteter Brennesselextrakt, wäßrig in Glykolen (4-5 % Festsubstanz; INCI-Bezeichnung: Aqua (and) Ethoxydiglycol (and) Propylene Glycol (and) Butylene Glycol (and) Nettle Extract/Urtica Dioica) (DRAGOCO) | |
| ¹⁵ 5-Ureidohydantoin (INCI-Bezeichnung: Allantoin) (MERCK) | |

### 4.2. Haarkur, auf dem Haar verbleibend

| | |
|---|---|
| Sepigel®305 | 3,00 |
| Euxyl®K 400 | 0,30 |
| Lipos®E 300 10% | 3,00 |
| Merquat®550 | 0,10 |
| Polymer JR®-400¹⁶ | 0,05 |
| Dow Corning®939 | 0,20 |
| Panthenol⁸ | 0,20 |
| α-Tocopherol | 0,20 |
| Wasser, entsalzt | ad 100 |

| | |
|---|---|
| ¹⁶ quaternierte Hydroxyethylcellulose (INCI-Bezeichnung: Polyquaternium-10) (AMERCHOL) | |

### 4.3. Haarkur, auf dem Haar verbleibend

| | |
|---|---|
| Sepigel®305 | 2,00 |
| Carbopol®940 | 0,40 |
| Natrosol®250 HR¹⁷ | 0,50 |
| Euxyl®K 400 | 0,30 |
| Neutrol®TE | 0,50 |
| Lipos®E 300 10% | 2,50 |
| Polymer JR®-400 | 0,10 |
| Dow Corning®939 | 0,30 |
| Dow Corning®Q2-5220¹⁸ | 0,50 |
| Extrapon®Brennessel Spezial | 0,30 |
| Wasser, entsalzt | ad 100 |

| | |
|---|---|
| ¹⁷ Hydroxyethylcellulose (INCI-Bezeichnung: Hydroxyethylcellulose) (HERCULES POWDER) | |
| ¹⁸ Silikon-Glykol-Copolymer (DOW CORNING) | |

### 4.4. Haarkur, abzuspülen

| | |
|---|---|
| Sepigel®305 | 4,00 |
| Natrosol®250 HR | 0,50 |
| Euxyl®K 400 | 0,30 |
| Lipos®E 300 10% | 3,50 |
| Merquat®550 | 0,10 |
| Polymer JR®-400 | 0,05 |
| Dow Corning®939 | 0,75 |
| Panthenol⁸ | 0,60 |
| α-Tocopherol | 0,10 |
| Antara®430¹⁹ | 0,50 |
| Wasser, entsalzt | ad 100 |

| | |
|---|---|
| ¹⁹ Vinylpyrrolidon-Styrol-Copolymer (ca. 40 % Aktivsubstanz in Wasser; INCI-Bezeichnung: Styrene/PVP Copolymer) (ISP) | |

### 4.5. Haarspülung, abzuspülen

| | |
|---|---|
| Carbopol®940 | 0,30 |
| Euxyl®K 400 | 0,30 |
| Neutrol®TE | 0,50 |
| Lipos®E 300 10% | 3,00 |
| Dow Corning®939 | 0,60 |
| Panthenol⁸ | 0,50 |
| α-Tocopherol | 0,10 |
| Wasser, entsalzt | ad 100 |

### 4.6. Haarspülung, abzuspülen

| | |
|---|---|
| Natrosol®250 HR | 1,00 |
| Jaguar®HP 120 | 0,50 |
| Euxyl®K 400 | 0,30 |
| Lipos®E 300 10% | 2,50 |
| Merquat®550 | 0,50 |
| Merquat®100²⁰ | 0,10 |
| Polymer JR®-400 | 0,05 |
| Dow Corning®Q2-5220 | 1,50 |
| Panthenol⁸ | 0,30 |
| Wasser, entsalzt | ad 100 |

| | |
|---|---|
| ²⁰ Poly(dimethyldiallylammoniumchlorid) (40 % Aktivsubstanz; INCI-Bezeichnung: Polyquaternium-6) (CHEMVIRON) | |

## Patentansprüche

1. Verwendung von Hydrogelbildnern in wäßrigen Mitteln mit liposomalen Strukturen zur Behandlung keratinischer Fasern.

2. Verwendung nach Anspruch 1, **dadurch gekennzeichnet, dass** es sich um einen anionischen Hydrogelbildner handelt.

3. Verwendung nach Anspruch 2, **dadurch gekennzeichnet, dass** es sich um ein Polymer mit Carboxylat- und/oder Sulfonatgruppen handelt.

4. Verwendung nach Anspruch 3, **dadurch gekennzeichnet, dass** es sich um ein Copolymer mit mindestens einem nichtionogenen Monomer handelt.

5. Verwendung nach Anspruch 4, **dadurch gekennzeichnet, dass** das nichtionogene Monomer ausgewählt ist aus Acrylamid, Methacrylamid, Acrylsäureestern, Methacrylsäureestern, Vinylpyrrolidon und Vinylestern.

6. Verwendung nach Anspruch 1, **dadurch gekennzeichnet, dass** der Hydrogelbildner ein nichtionogenes Polymer ist.

7. Verwendung nach Anspruch 6, **dadurch gekennzeichnet, dass** das nichtionogene Polymer ausgewählt ist aus Polyacrylamid, Polymethacrylamid sowie Copolymeren aus den genannten Monomeren.

8. Verwendung nach Anspruch 7, **dadurch gekennzeichnet, dass** das nichtionogene Polymer Polyacrylamid ist.

9. Verwendung nach einem der Ansprüche 1 bis 8, **dadurch gekennzeichnet, dass** die Liposomen aus Phospholipiden, Sphingolipiden und/oder Ceramiden aufgebaut sind.

10. Verwendung nach Anspruch 9, **dadurch gekennzeichnet, dass** die Liposomen aus Phospholipiden aufgebaut sind.

11. Verwendung nach Anspruch 10, **dadurch gekennzeichnet, dass** es sich um pflanzliche Phospholipide handelt.

12. Wäßriges Mittel mit liposomalen Strukturen zur Behandlung von keratinischen Fasern, **dadurch gekennzeichnet, dass** es
a) ein anionisches Polymer mit Sulfonatgruppen als Hydrogelbildner enthält, und
b) die Liposomen aus Phospholipiden, Sphingolipiden und/oder Ceramiden aufgebaut sind.

13. Mittel nach Anspruch 12, **dadurch gekennzeichnet, dass** es sich um pflanzliche Phospholipide handelt.

14. Mittel nach Anspruch 13, **dadurch gekennzeichnet, dass** das pflanzliche Phospholipid ausgewählt ist aus Sojalecithin, Rapslecithin, Erdnußlecithin, Sonnenblumenlecithin und Baumwollsaatöllecithin.

15. Mittel nach einem der Ansprüche 12 bis 14, **dadurch gekennzeichnet, dass** das Mittel eine Ölkomponente enthält.

16. Mittel nach Anspruch 15, **dadurch gekennzeichnet, dass** die Ölkomponente ein Kohlenwasserstoff, ein synthetisches Silikon oder ein natürliches pflanzliches oder tierisches Öl ist.

17. Mittel nach Anspruch 16, **dadurch gekennzeichnet, dass** es ein pflanzliches Öl, ausgewählt aus der Gruppe, die Weizenkeimöl, Mandelöl, Macadamianußöl, Sonnenblumenöl, Babassuöl, Avocadoöl, Olivenöl, Traubenkernöl und Nachtkerzenöl umfaßt, enthält.

18. Mittel nach einem der Ansprüche 12 bis 17, **dadurch gekennzeichnet, dass** das Mittel weiterhin eine haaravivierende Komponente enthält, die aus kationischen Polymeren, quaternären Ammoniumverbindungen und Silikonölen ausgewählt ist.

19. Mittel nach Anspruch 18, **dadurch gekennzeichnet, dass** als haaravivierende Komponente ein kationisches Polymer enthalten ist.

20. Mittel nach einem der Ansprüche 12 bis 19, **dadurch gekennzeichnet, dass** die Liposomen mit einem kosmetischen Wirkstoff beladen sind.

## Claims

1. The use of hydrogel formers in aqueous preparations containing liposomal structures for treating keratin fibers.

2. The use claimed in claim 1, **characterized in that** the hydrogel former is an anionic hydrogel former.

3. The use claimed in claim 2, **characterized in that** the hydrogel former is a polymer containing carboxylate and/or sulfonate groups.

4. The use claimed in claim 3, **characterized in that** the hydrogel former is a copolymer with at least one nonionic monomer.

5. The use claimed in claim 4, **characterized in that** the nonionic monomer is selected from acrylamide, methacrylamide, acrylates, methacrylates, vinyl pyrrolidone and vinyl esters.

6. The use claimed in claim 1, **characterized in that** the hydrogel former is a nonionic polymer.

7. The use claimed in claim 6, **characterized in that** the nonionic polymer is selected from polyacrylamide, polymethacrylamide and copolymers of the monomers mentioned.

8. The use claimed in claim 7, **characterized in that** the nonionic polymer is polyacrylamide.

9. The use claimed in any of claims 1 to 8, **characterized in that** the liposomes are made from phospholipids, sphingolipids and/or ceramides.

10. The use claimed in claim 9, **characterized in that** the liposomes are made from phospholipids.

11. The use claimed in claim 10, **characterized in that** the phospholipids are vegetable phospholipids.

12. An aqueous preparation containing liposomal structures for treating keratin fibers, **characterized in that**
a) it contains an anionic polymer containing sulfonate groups as the hydrogel former and
b) the liposomes are made from phospholipids, sphingolipids and/or ceramides.

13. A preparation as claimed in claim 12, **characterized in that** the phospholipids are vegetable phospholipids.

14. A preparation as claimed in claim 13, **characterized in that** the vegetable phospholipid is selected from soya lecithin, rape lecithin, peanut lecithin, sunflower lecithin and cottonseed oil lecithin:

15. A preparation as claimed in any of claims 12 to 14, **characterized in that** the preparation contains an oil component.

16. A preparation as claimed in claim 15, **characterized in that** the oil component is a hydrocarbon, a synthetic silicone or a natural vegetable or animal oil.

17. A preparation as claimed in claim 16, **characterized in that** it contains a vegetable oil selected from the group consisting of wheat germ oil, almond oil, macadamia nut oil, sunflower oil, babassu oil, avocado oil, olive oil, grapeseed oil and evening primrose oil.

18. A preparation as claimed in any of claims 12 to 17, **characterized in that** it additionally contains a hair-conditioning component selected from cationic polymers, quaternary ammonium compounds and silicone oils.

19. A preparation as claimed in claim 18, **characterized in that** it contains a cationic polymer as the hair-conditioning component.

20. A preparation as claimed in any of claims 12 to 19, **characterized in that** the liposomes are charged with a cosmetic active substance.

## Revendications

1. Utilisation d'agents de formation d'hydrogels dans des milieux aqueux avec des structures liposomales pour le traitement de fibres kératiniques.

2. Utilisation selon la revendication 1, **caractérisée en ce qu'**il s'agit d'un agent anionique de formation d'hydrogels.

3. Utilisation selon la revendication 2, **caractérisée en ce qu'**il s'agit d'un polymère comprenant des groupes carboxylate et/ou sulfonate.

4. Utilisation selon la revendication 3, **caractérisée en ce qu'**il s'agit d'un copolymère comprenant au moins un monomère non ionogène.

5. Utilisation selon la revendication 4, **caractérisée en ce que** le monomère non ionogène est choisi parmi l'acrylamide, le méthacrylamide, les esters de l'acide acrylique, les esters de l'acide méthacrylique, la vinylpyrrolidone et les esters de vinyle.

6. Utilisation selon la revendication 1, **caractérisée en ce que** l'agent de formation d'hydrogels est un polymère non ionogène.

7. Utilisation selon la revendication 6, **caractérisée en ce que** le polymère non ionogène est choisi parmi le polyacrylamide, le polyméthacrylamide ainsi que les copolymères des monomères mentionnés.

8. Utilisation selon la revendication 7, **caractérisée en ce que** le polymère non ionogène est le polyacrylamide.

9. Utilisation selon l'une quelconque des revendications 1 à 8, **caractérisée en ce que** les liposomes sont élaborés à partir de phospholipides, sphingolipides et/ou céramides.

10. Utilisation selon la revendication 9, **caractérisée en ce que** les liposomes sont élaborés à partir de phospholipides.

11. Utilisation selon la revendication 10, **caractérisée en ce qu'**il s'agit de phospholipides végétaux.

12. Agent aqueux avec des structures liposomales pour le traitement de fibres kératiniques, caractérisé
a) en ce qu'il contient un polymère anionique avec des groupes sulfonate comme agent de formation d'hydrogels et
b) en ce que les liposomes sont élaborés à partir de phospholipides, sphingolipides et/ou céramides.

13. Agent selon la revendication 12, **caractérisé en ce qu'**il s'agit de phospholipides végétaux.

14. Agent selon la revendication 13, **caractérisé en ce que** le phospholipide végétal est choisi parmi la lécithine de soja, la lécithine de colza, la lécithine d'arachide, la lécithine de tournesol et la lécithine de germes de coton.

15. Agent selon l'une quelconque des revendications 12 à 14, **caractérisé en ce que** l'agent contient une composante huileuse.

16. Agent selon la revendication 15, **caractérisé en ce que** la composante huileuse est un hydrocarbure, un silicone synthétique ou une huile végétale ou animale naturelle.

17. Agent selon la revendication 16, **caractérisé en ce qu'**il s'agit d'une huile végétale, choisie parmi le groupe comprenant l'huile de germe de blé, l'huile d'amande, l'huile de noix de macadamia, l'huile de tournesol, l'huile de babassu, l'huile d'avocat, l'huile d'olive, l'huile de pépin de raisin et l'huile d'onagre.

18. Agent selon l'une quelconque des revendications 12 à 17, **caractérisé en ce que** l'agent contient en outre un composant d'avivage des cheveux, qui est choisi parmi les polymères cationiques, les composés d'ammonium quaternaire et les huiles de silicone.

19. Agent selon la revendication 18, **caractérisé en ce qu'**un polymère cationique est contenu comme composant d'avivage des cheveux.

20. Agent selon l'une quelconque des revendications 12 à 19, **caractérisé en ce que** les liposomes sont chargés d'un agent actif cosmétique.
